# EUROPEAN PATENT APPLICATION

(11) **EP 2 138 458 A1**
(43) Date of publication of application: **30.12.2009**
(21) Application number: 09000481.3
(22) Date of filing: 19.04.2005
(51) Int. Cl.: C01B 31/30, C01B 31/36

(54) **High throughput discovery of materials through vapor phase synthesis**

(30) Priority: 19.04.2004 US 563853 P; 08.10.2004 US 617586 P
(62) Divisional of application: 05780032.8
(71) Applicant: SDC Materials, LLC, Tempe, AZ 85281 (US)
(72) Inventor: Pesiri, David R., Laguna Beach, CA 92651 (US); Walter, Kevin C., Aliso Viejo, CA 92656 (US)
(74) Representative: DeVile, Jonathan Mark

(57) **Abstract**

A method of and apparatus for producing a plurality of powder compositions, both stoichiometric and non-stoichiometric, is disclosed. The method includes the steps of introducing a plurality of materials into a particle production system under a first set of operating parameters, introducing the plurality of materials into the particle production system under a second set of operating parameters wherein at least one operating parameter of the first set is changed to form the second set, and sampling at least a portion of an output mixture representative of each of the first set of operating conditions and the second set of operating conditions. The apparatus includes a reactor, dispensing devices for supplying materials to the reactor, and other structures.; The apparatus is preferably configurable via modification of operating parameters to produce a range of possible compositions for a given plurality of input materials.

## Description

### Cross-Reference to Related Applications

This application claims priority under 35 U.S.C. §119(e) to co-pending Provisional United States Patent Application No. 60/563,853 filed April 19, 2004 and to co-pending Provisional United States Patent Application No. 60/617,586 filed October 8, 2004, both of which are hereby incorporated by reference.

### Field of the Invention

This invention relates to the field of powder material production and more specifically to a process of and apparatus for synthesizing, isolating and evaluating a spectrum of powders having a broad range of ingredients to find new powder compositions.

### Background of the Field of the Invention

New materials provide new possibilities; manufacturing techniques are so refined that in many cases improvement in end-product quality can only come about by fine control over the properties of manufacturing inputs. To find a material with precisely desired properties is a complex and expensive task, and a wide variety of emergent technologies and techniques have been developed to aid in new materials discovery.

Generally, a materials discovery process include production steps in which discovery production techniques are used produce a wide variety of composite outputs given certain material inputs, the outputs from the production steps are analyzed to determine which, if any, of the composite outputs possess desired properties. Following determination and analysis of desired properties, manufacturing production techniques are used to produce larger scale quantities of the desired material.

Modem materials discovery techniques include solution phase techniques, deposition techniques, furnace techniques, and sintering techniques. Because over one hundred periodic table elements can be reasonably combined into compounds consisting of several or more elements, the compounds known and considered useful constitutes a mere fraction of those possible. Hence, any materials discovery technology with a reasonable hope of success must provide for the rapid, systematic, repeatable, efficient, scalable, and economical production of a wide variety of material compositions. Traditional sintering and furnace techniques, in which solid phase particles or bulk portions of materials are physically combined and heated to react, fail to provide reasonable means to produce a wide variety of material compositions within a short period of time.

Solution phase techniques require strict temperature controls-reactants and products combined are generally soluble, and thus reactive, in a given solvent only within certain temperature and pressure ranges. Because acceptable ranges for common solvents do not even approach the melting points of many elemental metals, desired reactants must first be combined into soluble precursor compounds. The need for such precursor compounds adds complicating steps to the discovery process and restricts the list of candidate reactants to elements which can be combined into soluble compounds. Further, the expense of synthesizing or purchasing such compounds can be significant. Hence, commercial feasibility of large-scale solution phase synthesis of a material comprising an arbitrary set of reactants cannot be guaranteed.

Deposition techniques deliver multiple material reactants to a location and rely on an underlying means of synthesis to combine them into an array of materials. For example, a plurality of different reactant combinations are deposited to a substrate and then reacted with one another via solution or solid phase techniques or solid phase techniques to form a plurality of compounds comprising the reactants. Such a deposition based system is disclosed in U.S. Patent No. 5,776,359 to Schultz, et al., entitled "GIANT MAGNETORESISTIVE COBALT OXIDE COMPOUNDS," which is hereby incorporated by reference in its entirety. Because these techniques rely on deposited layers of material, which must be probed in order to analyze material properties, they are not suitable for bulk materials production.

Relatively recently, in part because nanostructured powders have proven their value to modem industry in a surprising variety of ways, capability to produce powders of very fine, and very even, grain size has been developed. A rich patent literature exists describing methods to produce high purity fine powders. One method of producing fine powders is described in U.S. Patent No. 6,786,950 to Yadav et al., entitled "HIGH PURITY FINE METAL POWDERS AND METHODS TO PRODUCE SUCH POWDER," which is hereby incorporated by reference in its entirety. Technological processes for production of fine powders generally fall into the broad categories of solution phase synthesis, mechanical synthesis, and vapor phase synthesis.

In solution phase techniques, aqueous, solubilized and/or suspended precursors react to form products which can remain dispersed in solution, or precipitate into solid or semisolid form. For example, synthesis can take place in a liquid solution, yielding precipitated solid powder. Alternately, a liquid solution reaction can yield a liquid solution product, which is then processed to yield a solid product in powder form. Similarly, in aerosol solution synthesis, precursors react within an aerosol solution and form products, which are processed to yield a desired powder. In another variety of solution phase synthesis, known as sol-gel, precursors are placed in liquid phase solution to form a colloidal solution, which is processed to yield a desired powder.

In mechanical synthesis techniques, material precursors are processed physically to form desired powders. Spraying techniques require the provision of liquid or vapor phase precursors or liquefaction of solid precursors. Sprayed precursors react with each other and/or with supplied atmospheres to produce powders. Milling techniques require the physical deconstruction of bulk quantities of precursor material into smaller powder grains.

Vapor or gas phase powder synthesis technologies include evaporative techniques, combustion techniques, plasma techniques, laser ablation techniques and vapor deposition techniques. In these techniques, reactions take place among gas or vapor phase reactants to produce gas or vapor phase particles. The produced particles are then processes to produce powders comprising the product material and having desired grain characteristics. Some gas phase techniques are amenable to a wider variety of reactant types than others. For example, evaporative techniques and certain vapor deposition techniques require provision of liquid or vapor phase precursor materials, ablation techniques rely on absorption of light energy by the working material, and combustion techniques generally operate at relatively low temperatures relative to melting and boiling points of common elemental metals. Though many materials can be provided in vapor phase compounds, or modified to exhibit required absorption characteristics, and reaction temperatures are adjustable to allow a broad range of reactants, combination of multiple reactants having drastically different physical properties via these methods is problematic. Combination of multiple reactants into a specified powder requires participation of each reactant in a vapor phase reaction; hence, each of these techniques cannot operate at temperatures where all known reactant forms are vaporous, and their purview is limited. A more inclusive option is synthesis based in plasma, where the temperatures of the reaction zone exceed the boiling points of even the most tightly bonded elemental materials.

Further, some of the above methods can be applied to produce non-stoichiometric powders. Non-stoichiometric materials and some known methods to produce them, are discussed in further detail in U.S. Patent No. 6,562,495 to Yadav et al., entitled "NANOSCALE CATALYST COMPOSITIONS FROM COMPLEX AND NON-STOICHIOMETRIC COMPOSITIONS," which is hereby incorporated by reference herein.

Because of the wide variety of powder production methods now known, a proliferation of materials discovery methods using powder production techniques is reasonable. Unfortunately, though powder production methods hold promise for materials discovery applications, current materials discovery methods adaptable to employ powder production techniques-such as the technique disclosed by the Schultz patent mentioned above-cannot deliver new materials in a reproducible and cost effective manner on a large scale, and may fail to deliver new materials in industrially valuable form.

Once a material is discovered through one of these processes, and its characteristics analyzed, a manufacturing scale production system must be configured to allow manufacturing scale production of the discovered material. Current materials discovery processes are separated from manufacturing processes, and suffer from non-scalable bottlenecks which prevent the production methods used therein from scaling efficiently to manufacturing production levels.

The current materials discovery systems that employ powder production technologies fail to delivery new material candidates in useful form. Candidate materials supplied in forms without recognized use in industry must be reprocessed into a useful form; alternately, useful forms of the candidate material can be produced via separate processes. In either case, the failure of current materials discovery systems to provide candidate materials in versatile, industrially valuable forms-such as fine powder form-prevents efficient testing and confirmation of candidate materials.

### Summary of the Disclosure

In contrast to the prior art, the present invention presents a materials discovery system employing a high throughput gas phase powder production systeni to produce a broad range of powder compositions from a readily adjustable set of material inputs, and a sampling system to sample and analyze selected powder compositions. The array of powder compositions produced includes both stoichiometric and non-stoichiometric compounds of the material inputs. Preferably, powder production systems employed within the present invention achieve non-stoichiometric compositions thorough some combination of high throughput reactions, controlled reactant introduction rates and high quench rates which promote formation of kinetically stable-and possibly thermodynamic metastable-compounds. This capability of the production systems to form non-stoichiometric outputs under thermodynamic non-equilibrium does not preclude production of stoichiometric outputs under thermodynamically preferred conditions within these same systems.

Powders produced within the given invention can include any of the following: "nanostructred powders," having an average grain size less than 250 nanometers and an aspect ratio between one and one million; "submicron powders," having an average grain size less than 1 micron and an aspect ratio between one and one million; "ultra-fine powders," having an average grain size less than 100 microns and an aspect ratio between one and one million; and "fine powders," having an average grain size less than 500 microns and an aspect ratio between one and one million.

As will be described in more detail hereinafter, there is disclosed herein a method of forming a plurality of material compositions. The method includes the steps of introducing a plurality of materials into a particle production system under a first set of operating parameters, introducing the plurality of materials into the particle production system under a second set of operating parameters wherein at least one operating parameter of the first set is changed to form the second set; and, sampling at least a portion of each of a first output mixture representative of the first set of operating parameters and a second output mixture representative of the second set of operating parameters.

Preferably, the method includes further steps to facilitate repeatability. A further step of correlating the sampled portions with the individually selected rates forms a relationship between each material produced and the conditions under which it is produced. Further, a step of assaying the sampled portions to discover material properties, combined with the correlation of portions with rates, allows correlation desired material properties with individually selected rates. Hence, a step of correlating properties of the plurality of sampled portions with a set of values for the set of operating parameters and with at least one of the plurality of periods of time is preferably also included. Forming a known relationship between production parameters and product properties allows repeatable production of materials having desired properties.

In the method of materials discovery of the present invention, the set of operating parameters can be large; preferably, at least one parameter is changed to form the second set. Operating parameters include individually selected rates of introduction of the plurality of materials, type of precursor material, ratio of the amount introduced of each material of the plurality of materials, relative location of introduction, system energy, throughput rate, and other operating parameters. Because not every parameter need vary, in one aspect only an operating parameter of the first set of operating parameters relating to a ratio of materials within the plurality of materials introduced is changed to form the second set of operating parameters. In a further aspect, only operating parameters relating to system energies are changed, in further aspect, only operating parameters relating to rates of introduction are changed.

Also included in the present invention is a method of producing a plurality of powder compositions. The method comprises the steps of introducing at a first selectable rate a first material into a gas phase particle production reactor under a set of operating parameters, and of introducing at a second selectable rate and substantially simultaneously, a second material into the gas phase particle production reactor to produce a first powder having a first composition.

Preferably, the method of the present invention is repeated, with varying parameters. That is, the values of one or more of the first selectable rate, the second selectable rate, the first material, the second material, and the set of operating parameters are modified, and the steps of 'introducing at a first selectable rate' and 'introducing at a second selectable rate' are repeated to produce a second powder having a second composition.

In a further aspect, the method involves a plurality of materials. The plurality of materials are introduced at a set of rates into the gas phase particle production reactor. Preferably one individually determined rate corresponds to each material of the plurality. The rates of the set of rates are understood to be independent of one another, and no particular relationship between the rates is necessary to practice the present invention. However, though no correlation between the rates is necessary, the rates are preferably varied in a manner to yield a broad spectrum of material compositions. Alternatively the materials can be varied. In a further aspect, the operating parameters can be varied. Following each variation, the steps of 'introducing at a first selectable rate,' 'introducing at a second selectable rate' and 'introducing at a set of rates' are repeated. Varying or changing a rate or parameter is understood to mean a modification of the value of the rate or parameter, whereas varying or changing a material is understood to mean a substitution of a different material, or a different physical form of the same material. In another aspect, the set of operating parameters is changed and the steps of 'introducing at a first selectable rate', 'introducing at a second selectable rate' and 'introducing at a set of rates' are repeated.

Preferably, at least a portion of each of the powders produced are sampled. In the aspects of the present invention where the above process is repeated, while varying one or more parameters, powders having other compositions are produced. Preferably these powders are also sampled.

A sampled portion of the powder having the first composition is preferably used to correlate properties of powders having the first composition with the first selectable rate of the first material and the second selectable rate of the second material. An exemplary correlation method is to track the first selectable rate and the second selectable rate and correlate it to the portion sampled from the output. Then any reasonable assay can be used to determine properties the first composition from that portion. Hence, the first composition need not be known to correlate its properties to the first and the second selectable rates. Knowledge of the first and the second selectable rates and the set of operating parameters allows repeated synthesis of powder having the first composition and thus the determined properties, according to the method of the present invention.

In a further aspect of the present invention, a method of synthesizing a plurality of materials and sampling the materials is provided. The method includes two sets of similar steps, one set performed during each of a first production time and a second production time.

According to the present invention, during the first production time a step of introducing a plurality of materials each at a first individually selected rate into a powder production reactor having a first set of operating parameters is performed. Preferably, each of the plurality of materials is a member of a set of possible constituent ingredients. Also during the first production time a step of sampling at least a portion of a first output-mixture of the powder production reactor, the first output mixture comprising one or more members of the set of constituent ingredients present in a first ingredient ratio is performed.

During the second production time the following steps are performed: introducing the plurality of materials into the powder production reactor having a second set of operating parameters, each at a second individually selected rate; and sampling at least a portion of a second output mixture of the powder production reactor, the second output mixture comprising one or more members of the set of constituent ingredients present in a second ingredient ratio.

In the present invention, one or more parameters can be substantially the same during both the first and the second production times. In one aspect, the first and the second sets of operating parameters are substantially the same. In another aspect, the first and the second individually selected rates are each substantially the same. In a further aspect, the second individually selected rates differ from the first individually selected rates only in the individually selected rate of a subset of the plurality of materials.

Preferably, the method of the present invention further comprises repeating the steps of 'introducing' through 'sampling' during n subsequent production times, wherein each ith production time, where i is an integer less than or equal to n, is associated with an ith set of operating parameters, and an ith set of individually selected rates. For each ith production time, an ith output mixture is produced. Depending on the individually selected rates and the set of operating parameters, the ith output mixture for a given i may differ from the output mixtures for all other i. The method presented is capable of producing a unique output mixture for each production time within a given set of production times. The repeated introduction of the plurality of materials with a variety of sets of operating conditions, forms a range of samples.

Further, the duration of the first and the second production times are preferably long enough that a quasi-steady state is present in the first output mixture and the second output mixture. This limitation preferably also is applied if the process has a plurality of production times, so that for each production time a quasi-steady state is established in the output mixture at some time during the production time.

In a continuing aspect, the present invention is directed to a process for high throughput production of a set of distinct materials from a group of precursor elements. This process preferably takes place during a plurality of production times. During a first production time the following steps are performed: introducing a first material input at a first selectable rate into a particle production reactor having a first set of operating parameters; introducing a second material input into the particle production reactor at a second selectable rate; and, sampling at least a portion of an output mixture of the particle production reactor. During a second materials production time the steps of introducing a first material, introducing a second material, and sampling are repeated using a second set of operating parameters, a third selectable rate of introduction of the first material, and a fourth selectable rate of introduction of the second material. During further production times, the same steps of 'introducing' through 'sampling' are repeated and during each production time an independent set of operating parameters and two independent rates are used, one for the first material and one for the second material to form a desired number of distinct materials.

This method involves several parameters: set of operating parameters, a rate of introduction of the first material, and a rate of introduction of a second material. Preferably, only a single parameter is changed during each materials production time. Specifically, in one aspect the first set of operating parameters and the second set of operating parameters are substantially the same. In another aspect, the first selectable rate and the third selectable rate are the same. In a further aspect the second selectable rate and the fourth selectable rate are the same.

In a further aspect, the method comprises introducing additional materials, each at an independent rate. Preferably, these additional materials are introduced substantially simultaneously with the first and second materials.

Further, a materials discovery system is presented. The materials production system comprises a particle production system having a reaction chamber and an output port to conduct an output mixture from the reaction chamber, a plurality of dispensing devices for supplying materials to the reaction chamber at selectable rates, a sampling device coupled with the output port to selectively sample portions of the output mixture, and a controller coupled with the plurality of input material dispensing devices, with the particle production system, and with the sampling device, and configured to modify the selectable rates of the plurality of dispensing devices, to control operating parameters of the powder production system, and to control selective sampling of portions of the output mixture.

An exemplary apparatus for synthesizing powders is also presented in accordance with the present invention. The apparatus comprises a plasma reactor, a plurality of dispensing devices coupled with the plasma reactor for introducing materials into the plasma reactor, and at least one sampling device, coupled with the plasma reactor and configured to selectively sample portions of an output mixture.

Preferably, the plasma reactor has a plurality of ports for introducing materials into the plasma reactor, at least one port for supplying a working gas to the plasma reactor, at least one output port, and an energy delivery system to deliver a selectable energy to a plasma within the plasma reactor. Further, the dispensing devices are preferably coupled with the plurality of ports. In addition, the sampling device, is preferably coupled with the output port.

The sampling device is coupled with the output port via a conduit configured to cool and conduct the output mixture to a bulk materials container. Preferably, the sampling occurs during transport of the output mixture. Furthermore, the sampling device is preferably capable of selectively sampling portions of multiple output mixtures sequentially without cross-contamination between the multiple output mixtures.

In a further aspect, the present invention includes an exemplary materials production system. The materials production system includes a plasma reactor to produce an output mixture having a reaction chamber, a plurality of dispensing devices for supplying materials to the reaction chamber at selectable rates, a sampling device coupled with the output port to selectively sample portions of the output mixture, and a controller coupled with the plurality of input material dispensing devices, with the plasma reactor and with the sampling device.

The plasma reactor preferably defines a path passing through the reaction chamber. One end of the path is coupled with an input port for supplying a working gas to the reaction chamber, and one end of the path is coupled with an output port to conduct an output mixture from the reaction chamber. In addition, the controller is preferably configured to modify the selectable rates of the plurality of dispensing devices, to control selective sampling of portions of the output mixture, to control a plasma energy of a plasma within the plasma reactor, and to control other operating parameters.

### Description of the Several Drawings

Figure 1 is a schematic illustration of an apparatus for synthesizing powders incorporating the present invention.
Figure 2 is a more detailed illustration of a materials discovery system in accordance with the present invention.
Figure 3A and 3B are two graphs illustrating an exemplary sequence of varied materials introduction rates and the broad spectrum of material compositions produced thereby in accordance with the present invention.
Figure 4 is a flowchart illustrating a method of producing a plurality of powder compositions in accordance with the present invention.
Figure 5 is a flowchart illustrating a method of synthesizing a plurality of materials and sampling the materials in accordance with the present invention.
Figure 6 is a flowchart illustrating a method of forming a plurality of material compositions in accordance with the present invention.

### Detailed Description

The following description is presented to enable one of ordinary skill in the art to make and use the invention and is provided in the context of a patent application and its requirements. Various modifications to the described embodiments will be readily apparent to those skilled in the art and the generic principles herein may be applied to other embodiments. Thus, the present invention is not intended to be limited to the embodiment shown but is to be accorded the widest scope consistent with the principles and features described herein.

The present invention considers a wide variety of gas phase particle production systems including combustion based systems, plasma based systems, laser ablation systems and vapor deposition systems. The preferred gas phase particle production systems employed within the present invention are capable of performing high throughput reactions between a large number of reactants at selectable quench rates. Control over the throughput and quench rate of the production process permits production of a broad range of kinetically and thermodynamically stable and metastable product compositions, including stoichiometric and non-stoichiometric compounds. Further, preferred systems take material inputs in a broad range of forms, including solid phase inputs, and provide product in high surface area forms, including powders ranging in grain size from nanostructured to fine. In addition, the process controls preferably provide a fine degree over a plurality of reaction parameters, permitting fine gradients of product composition ratios to be produced.

A wide variety of material types and forms can be processed in preferable particle production reactors used in the present invention. Without prejudice, the present invention specifically considers the provision of materials in the following forms: solid, liquid and gas.

An exemplary particle production system is a plasma powder production reactor, which is included within several of the exemplary embodiments discussed below. The plasma reactors considered within the present invention can have many means of energy delivery, including the following: DC coupling, capacitive coupling, inductive coupling, and resonant coupling.

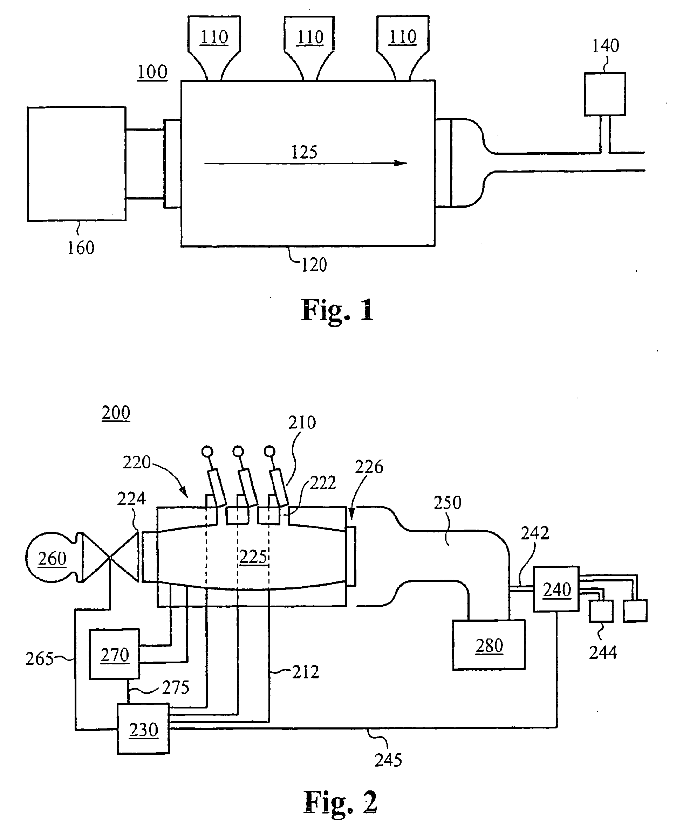

Referring now to FIG. 1, the apparatus 100 comprises a plasma reactor 120, a plurality of dispensing devices 110, and at least one sampling device 140. In the present invention, the dispensing devices 110 are coupled with the plasma reactor 120 and configured for introducing materials into the plasma reactor 120. The sampling device 140 is coupled with the plasma reactor 120 Still referring to FIG. 1, the operation of the apparatus 100 preferably occurs as follows. Working gas is supplied from the gas source 160 to the plasma reactor 120. Within the plasma reactor 120, energy is delivered to the working gas, creating a plasma. The materials dispensing devices 110 each introduce at least one material into the plasma reactor 120. The combination within the plasma reactor of the plasma and the plurality of materials introduced by the materials dispensing devices 110 forms a highly reactive and energetic mixture. This mixture moves through the plasma reactor 120 in the flow direction of the working gas, illustrated by the arrow 125. As it moves, the mixture cools and particles are formed therein. This still-energetic output mixture, comprising hot gas and energetic particles, is emitted from the plasma reactor 120. Following emission from the plasma reactor 120, the output mixture cools further and is exposed to the sampling device 140, which selectively samples portions of the output mixture. According to the present invention, sampling occurs during transport of the output mixture from the plasma reactor 120, while the output mixture comprises hot gas and particles of relatively homogeneous size distribution.

In accordance with the present invention, the plasma reactor 120 is controlled to produce a series of quasi-steady state output mixtures, each containing particles substantially homogeneous in both their size distribution and their composition. The specific compositions and size distributions of the particles differ from one quasi-steady state output mixture to the next. The quasi-steady state output mixtures correspond to specific plasma energies, working gas compositions, material types, and rates of introduction of materials by the dispensing devices 110. A variety of output mixtures are produced by varying these parameters. In the present invention, the establishment of a quasi-steady state output mixture is followed by the sampling of that mixture and, more preferably, the isolation and analysis of the sample. The method of operating the apparatus 100 is described more thoroughly elsewhere in the disclosure of the present invention.

Referring now to FIG. 2, the materials production system 200 is illustrated. The materials production system 200 includes a plasma reactor 220 having a reaction chamber 225, a plurality of dispensing devices 210, a sampling device 240, and a controller 230 coupled with the plurality of input material dispensing devices 210 and with the sampling device 240.

The plasma reactor 220 defines a path passing through the reaction chamber 225. One end of the path is coupled with an input port 224 for supplying a working gas from a working gas supply 260 to the reaction chamber 225. The other end of the path is coupled with an output port 226 to conduct an output mixture from the reaction chamber 225. A plurality of ports 226 open into the reaction chamber 225 and the dispensing devices 210 are coupled with the plurality of ports 222 and therethrough can introduce materials into the reactor chamber 225. The energy delivery system 270 is coupled with the plasma reactor 220 and configured to deliver a selectable energy to a plasma within the reactor chamber 225. Exemplary plasma reactors include those using the following types of plasmas: constricted arc, free-burning electric arc, combustion flame, low pressure arc, and glow discharge. Exemplary energy delivery means include high intensity (DC and AC), RF, inductively coupled, microwave, and hybrid. Of course, other types of plasmas and energy delivery means are considered within the scope of the present invention.

The plurality of dispensing devices 210 are configured to accept bulk material input in a variety of forms. Though many forms are contemplated, the preferred forms include those which are both easily and economically obtained and are easily meterable, including, but not limited to, wires, powders, liquids, and gasses. Preferably, the devices incorporate a control interface (not shown) for selecting introduction parameters such as rate, volume and duration. Suitable interfaces include direct user interfaces, external controller interfaces (such as IEEE 488) and combinations thereof. The dispensing devices 210 can be chosen from any device capable of metering material input; however, a few types of devices are preferred. Such devices include the following devices calibrated to introduce a selectable amount of material into the plasma reactor: wire feeds, hoppers, and metering pumps.

The materials production system 200 further includes a conduit 250 aligned with the output port 226 of the plasma reactor 220. The working gas flowing through the plasma reactor 220 is conducted from the output port 226 through the conduit 250, which is configured to cool and conduct the output mixture to a bulk materials container 280, preferably via a vacuum system (not shown). Coupled with the conduit 250 is the sampling device 240, configured to selectively sample portions of the output mixture.

The sampling device 240 preferably comprises a sample tube 242 coupled with the conduit 250. The sample tube 242 is configured so that it encounters the output mixture flowing through the conduit 250. The sampling device 240 is capable of forming a sealable path from the opening of the sample tube 242 to a selectable one of the sample containers 244.

The controller 230 is configured to control several parameters during operation of the materials production system 200. The control system 230 is coupled with the dispensing devices 210 through the control lines 212, with the energy delivery system 270 through the control wire 275, with the working gas supply 260 through the control wire 265 and with the sampling device 240 through the control wire 245. Through the various control wires, the controller 230 controls the rates of material input of the dispensing devices 210, the amount of energy delivered to the plasma by the energy delivery system 270, the selective sampling of portions of the output mixture by the sampling device 240, and the supply of working gas to the plasma reactor 220 by the working gas supply 260.

Still referring to FIG. 2, the operation of the materials production system 200 preferably occurs as follows. Working gas is supplied from the gas source 260 to the plasma reactor 220, flowing through the input port 224 into the reaction chamber 225. Within the plasma reactor 225, energy is delivered from the energy delivery system 170 to the working gas within the reaction chamber 225, creating a plasma. The materials dispensing devices 210 each introduce at least one material into the reaction chamber 225 through one of the ports 222. The plasma formed within the reactor chamber 225 from the working gas, having very high thermal energy, easily incorporates the materials introduced from the plurality of dispensing devices 210 into the rector chamber 225. The combination within the reaction chamber 225 of the plasma and the plurality of materials introduced by the materials dispensing devices 210 forms a highly reactive and energetic mixture. This mixture moves through the reaction chamber 225 towards the output port 226. As the mixture moves toward the output port 226 the mixture cools and particles are formed. This still-energetic output mixture, comprising hot gas and energetic particles, is emitted from the plasma reactor 220 through the output port 226. Following emission from the output port 226, the output mixture is conducted through and cooled by the conduit 250. The output mixture is exposed to the sampling device 240, which selectively samples a portion there of by drawing it into the sample tube 242 and depositing it into a sample container 244. The sampling occurs during transport of the output mixture from the plasma reactor 220 to the bulk materials container 280, while the output mixture comprises hot gas and particles of relatively homogeneous size distribution.

In accordance with the present invention, the plasma reactor is controlled to produce a series of quasi-steady state output mixtures, each containing particles substantially homogeneous in both their size distribution and their composition. The quasi-steady state output mixtures vary in the specific composition and size distribution of their particles. The quasi-steady state output mixtures correspond to specific plasma energies supplied by the energy delivery device 270, working gas compositions and flow rates supplied by the working gas supply 260, material types and rates of introduction of materials by the dispensing devices 210. A variety of output mixtures are produced by varying these parameters.

The controller 230 selects a plurality of parameters and controls the dispensing devices 210, the energy delivery device 270, and the working gas supply 260 to produce a given output mixture. Upon establishment of a quasi-steady state output mixture, the controller 230 selectively samples a portion a portion of the output mixture. The sampling device 240 is controlled by the controller 230 to form a suction within the sample tube 242 capable of sampling a portion from the output mixture moving through the conduit 250 and delivering the portion to one of the sample containers 244. Preferably, the isolation and analysis of the sample follows. Once a sample of a given quasi-steady state output mixture is obtained, the above-mentioned parameters are changed by the controller 230 and a new quasi-steady state output mixture is established and sampled. Preferably, the sampling device 240 is capable of sampling multiple output mixtures sequentially without cross-contamination between the multiple output mixtures. The method of operating the materials production system 200 is described more thoroughly elsewhere in the disclosure of the present invention.

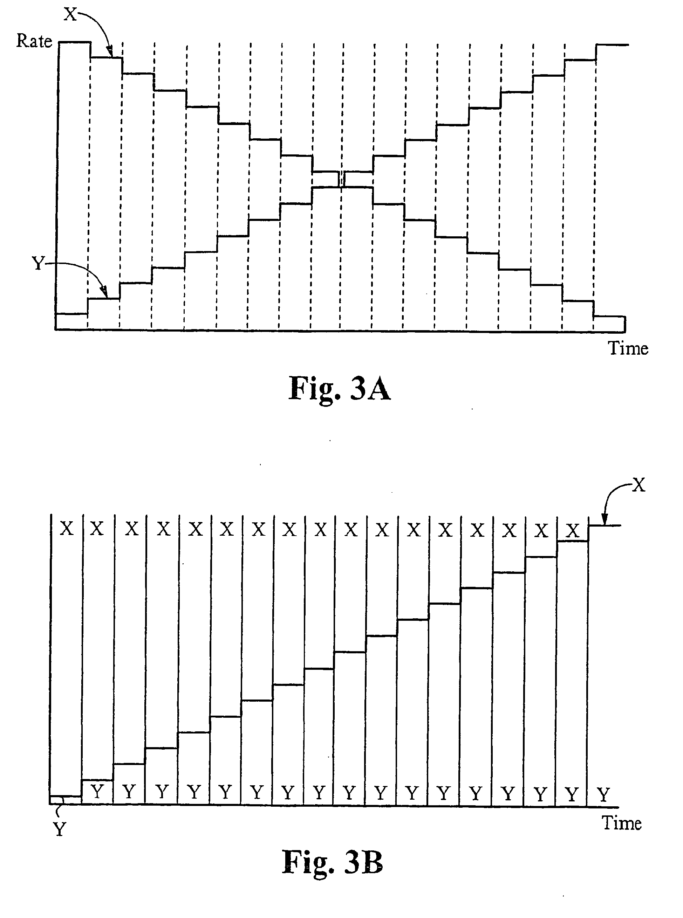

Referring now to FIG. 3, the outcome of operating a device in accordance with the present invention is discussed. As described above, the present invention includes varying a plurality of parameters affecting the production of powders within a particle production reactor to achieve quasi-steady state output form the particle production reactor, and simultaneously controlling the sampling of that output to obtain substantially homogeneous samples of a broad spectrum of materials, each having a varied composition ratio, which are substantially homogeneous in both particle size and composition. Though, as discussed above, many possible parameters are varied to achieve this end, the exemplary data shown in FIG. 3 considers only the variation of introduction rates of materials into a powder production reactor.

FIG. 3 presents a pair of graphs, illustrating corresponding sequences of input rates and output materials of a particle production reactor. The two graphs are coincident in time, FIG. 3A illustrates the rate of material input over time for two materials, X and Y, while FIG. 3B illustrates the idealized composition ratio of material output over the same time period, in terms of its percentage content of both X and Y. Referring now to FIG. 3A, note that the rates of material introduction are varied in a quasi-stepwise fashion, remaining constant over short periods of time, termed "steps." During each "step" an individual rate of introduction for both X and Y are used, as illustrated (on a relative scale). These rates correspond to a output material composition, as illustrated in FIG. 3B, and further explained below. In accordance with the present invention, by varying the introduction rates of both X and Y as illustrated and by sampling, a broad spectrum of materials having differing compositions is produced. This methodology is easily applied to a greater number of materials and corresponding feed rates, as contemplated by the present invention.

Though the material compositions in FIG. 3B are illustrated as covering the virtual entirety of each "step," the materials they represent are sampled only in one small subperiod. This methodology explains the homogeneity illustrated for the following reasons. It is recognized that the establishment of a quasi-steady state output occurs quickly in the type of reactor contemplated, given the conditions of steady state input rate, working gas flow rate, and energy supply rate. Thus, the sampling of materials from the output near the vertical lines of FIG. 3B, which can be though of as the "ends" of the steps, when the input feed rates have been constant over virtually the entire step time, will yield relatively homogeneous material output.

Further, though fairly large and discrete 'steps' are illustrated in FIGS. 3A and 3B, two aspects of the stepwise nature of the process bear discussion. First, the production times represented by each step can be shortened to instantaneous lengths. Because process control and sampling are coordinated, process parameters and output characteristics can be correlated for very short step times. Longer step times result in increased homogeneity of the product composition and thus ensure accurate correlation between production conditions and product compositions; however, overly long step times result in unnecessarily slow processes. Second, the variation between product compositions produced within adjacent steps is variable and termed the "gradient" of a given process. Greater differences in composition between adjacent step times allow coverage of a broader range of compositions within a fixed time period (for a fixed step size) but omits certain compositions from production. Thus, fine control of process parameters permits rapid production of an array of particle compositions having only slight differences, and allows more rapid identification of valuable and interesting compositions.

By adjusting both the step time and the gradient between compositions produced during adjacent steps, the process of the present invention provides a flexible discovery process. For example a short step time combined with a coarse gradient can be used to quickly scan a broad range of possible compositions. Following such a scan, possible candidate compositions can be identified and a second scan using a slightly longer step time combined with a finer gradient performed over a range of compositions in the neighborhood of the candidate compositions. The longer step time and finer gradient will produce greater quantities of candidate compositions within the neighborhood and permit more accurately identification of the preferred composition.

In a further aspect, process having very fine gradient and very short step time can be used to scan a range of compositions. Further processes can examine regions of the range of compositions in more detail, using longer step times to more accurately correlate preferred material characteristics with material composition.

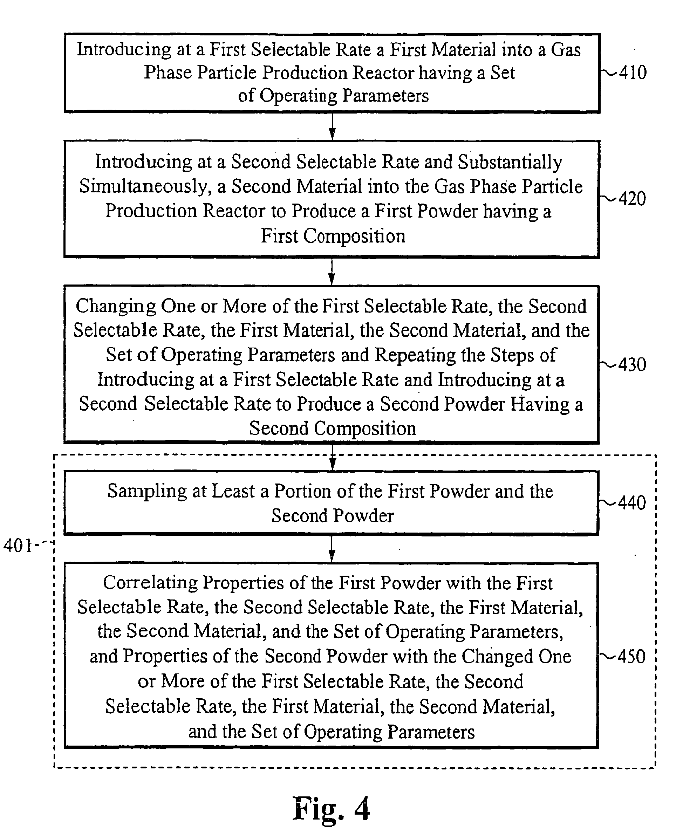

Referring now to FIG. 4, a method of producing a plurality of powder compositions is presented. The method comprises the steps 410 of introducing at a first selectable rate a first material into a particle production reactor having a set of operating parameters, and 420 of introducing at a second selectable rate and substantially simultaneously, a second material into the particle production reactor to produce a first powder having a first composition. In a further step the process of the present invention is repeated, with varying parameters: the step 430 includes changing one or more of the first selectable rate, the second selectable rate, the first material, the second material, and the set of operating parameters and repeating the steps 410 of introducing at a first selectable rate and 420 of introducing at a second selectable rate to produce a second powder having a second composition.

In additional steps 401, repetition, sampling and correlation occur. The additional step 440 includes sampling at least a portion of the first powder having the first composition and the second powder having the second composition. In the aspects of the present invention where the above process is repeated, while varying one or more parameters, powders having other compositions are produced and sampled.

The step 450 of correlating properties of the first powder with the first selectable rate, the second selectable rate, the first material, the second material, and the set of operating parameters, and properties of the second powder with the changed one or more of the first selectable rate, the second selectable rate, the first material, the second material, and the set of operating parameters. is preferably included. An exemplary correlation method is to track the first selectable rate and the second selectable rate and correlate it to the portion sampled from the output. Then any reasonable assay may be used to determine properties the first composition from that portion. Hence, the first composition need not be known to correlate its properties to the first and the second selectable rates. Knowledge of the first and the second selectable rates and possibly additional parameters allows repeated synthesis of powders having the first composition and thus the determined properties, according to the method of the present invention.

In a further aspect, the method is extended to encompass a production method using more than two types of materials. Preferably one individually determined rate corresponds to each material, and a step is performed for introducing each material at the individually determined rate. The rates of the set of rates are understood to be independent of one another, and no particular relationship between the rates is necessary to practice the present invention. However, though no correlation between the rates is necessary, the rates are preferably varied in a manner to yield a broad spectrum of material compositions through repetition of the process.

One or more of the rates, and the materials are varied; and the steps of 'introducing' are repeated. Varying a rate is understood to mean a modification of the value of the rate, whereas varying a material is understood to mean a substitution of a different material, or a different physical form of the same material. In another aspect, the set of operating parameters is changed and the steps of 'introducing' are repeated.

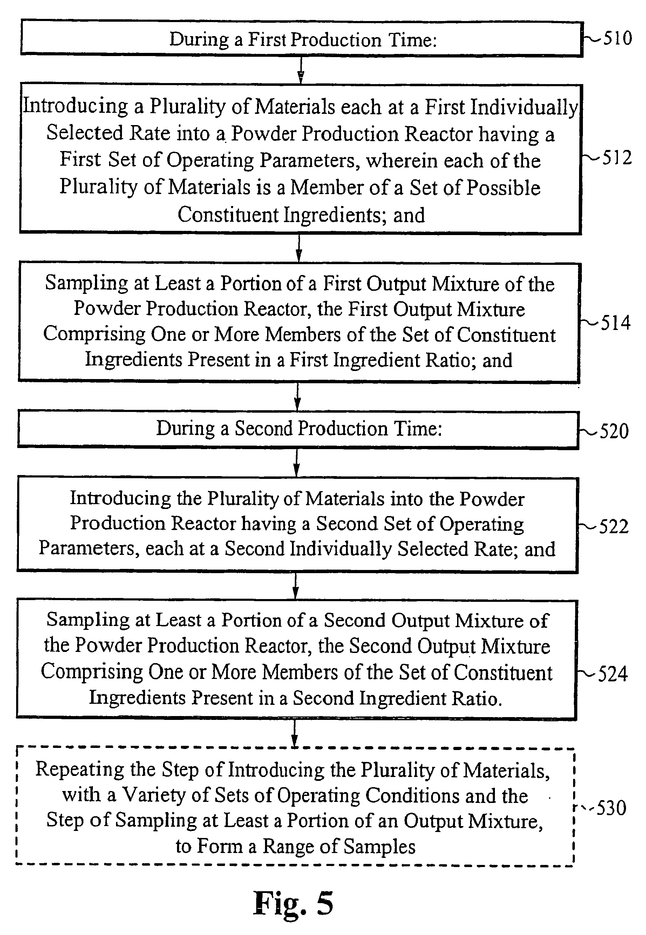

Referring now to FIG. 5, a method of synthesizing a plurality of materials and sampling the materials is provided. The method includes two sets of similar steps, the set 510 performed during a first production time and the set 520 performed during a second production time.

According to the present invention, the set 510 includes the substep 512 of introducing a plurality of materials each at a first individually selected rate into a powder production reactor having a first set of operating parameters. Preferably, each of the plurality of materials is a member of a set of possible constituent ingredients. The set 510 further includes the substep 514 of sampling at least a portion of a first output mixture of the powder production reactor, the first output mixture comprising one or more members of the set of constituent ingredients present in a first ingredient ratio.

The set 520 includes the substeps 522 of introducing the plurality of materials into the powder production reactor having a second set of operating parameters, each at a second individually selected rate; and 524 of sampling at least a portion of a second output mixture of the powder production reactor, the second output mixture comprising one or more members of the set of constituent ingredients present in a second ingredient ratio.

In the present invention, one or more parameters may be substantially the same during both the first and the second production times. In one aspect, the first and the second set of operating parameters are substantially the same. In another aspect, the first and the second individually selected rates are each substantially the same. In a further aspect, the second individually selected rates differ from the first individually selected rates only in the individually selected rate of a subset of the plurality of materials.

Preferably, the method of the present invention further comprises the step 530 of repeating the step of introducing the plurality of materials, with a variety of sets of operating conditions, and the step of sampling at least a portion of an output mixture, to form a range of samples. Alternative embodiments include a step of repeating the steps of introducing the plurality of materials the steps of 'introducing' through 'sampling' during n subsequent production times, wherein each ith production time, where i is an integer less than or equal to n, is associated with an ith set of operating parameters, and an ith set of individually selected rates. For each ith production time, an ith output mixture is produced. Depending on the individually selected rates and the set of operating parameters, the ith output mixture for a given i may differ from the output mixtures for all other i. The method presented is capable of producing a unique output mixture for each production times within a given set of production times.

Further, in the duration of the first and the second production times are preferably long enough that a quasi-steady state is present in the first output mixture and the second output mixture. This limitation preferably also is applied if the process has a plurality of production times, so that for each production time a quasi-steady state is established in the output mixture at some time during the production time.

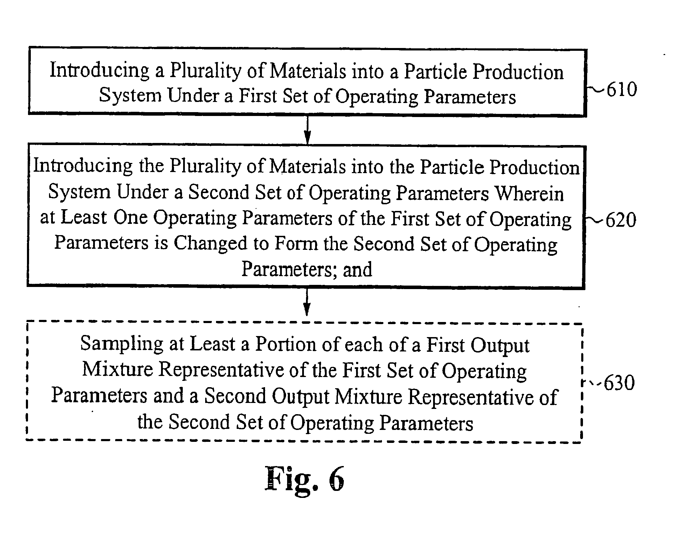

Referring now to FIG. 6, a method of forming a plurality of material compositions is presented. The method includes the steps 610 of introducing a plurality of materials into a particle production system under a first set of operating parameters, 620 of introducing the plurality of materials into the particle production system under a second set of operating parameters wherein at least one operating parameter of the first set is changed to form the second set, and, 630 of sampling at least a portion of each of a first output mixture representative of the first set of operating parameters and a second output mixture representative of the second set of operating parameters, to form a range of samples.

In the method of materials discovery of the present invention, a multiplicity of parameters can be changed. Preferably, at least one parameter is changed. These parameters can include the following: individually selected rates of introduction of the plurality of materials, type of material included in the plurality of materials, relative location of introduction of the plurality of materials, system energy, and reaction throughput rate. Because not every parameter need vary only operating parameters relating to rates of introduction of materials are changed in one embodiment, in another aspect, only operating parameters relating to system energies are changed.

The method can include further steps to facilitate repeatability. A step of correlating the sampled portions with a specific set of values for the set of operating parameters forms a relationship between each material produced and the conditions under which it is produced. Further, a step of assaying the sampled portions to discover material properties, combined with the correlation of portions with parameters, allows correlation desired material properties with values of the operating parameters. Hence, a step of correlating properties of the plurality of sampled portions with a set of values for the set of operating parameters and with at least one of the plurality of periods of time is preferably also included. Forming a known relationship between production conditions and product properties allows repeatable production of materials having desired properties.

The present invention has been described in terms of specific embodiments incorporating details to facilitate the understanding of the principles of construction and operation of the invention. As such, references herein to specific embodiments and details thereof are not intended to limit the scope of the claims appended hereto. It will be apparent to those skilled in the art that modifications can be made to the embodiments chosen for illustration without departing from the spirit and scope of the invention.

## Claims

1. A method of producing a plurality of powder compositions, comprising:
a. introducing at a first selectable rate a first material into a gas phase particle production reactor having a set of operating parameters;
b. introducing at a second selectable rate and substantially simultaneously, a second material into the gas phase particle production reactor to produce a first powder having a first composition; and,
c. changing one or more of the first selectable rate, the second selectable rate, the first material, the second material, and the set of operating parameters and repeating the steps of introducing at a first selectable rate and introducing at a second selectable rate to produce a second powder having a second composition.

2. The method of Claim 1, further comprising a step of sampling at least a portion of the first powder and the second powder.

3. The method of Claim 2, further comprising a step of correlating properties of the first powder with the first selectable rate, the second selectable rate, the first material, the second material, and the set of operating parameters, and properties of the second powder with the changed one or more of the first selectable rate, the second selectable rate, the first material, the second material, and the set of operating parameters.

4. A method of synthesizing a plurality of materials and sampling the materials, comprising:
a. during a first production time:
i. introducing a plurality of materials each at a first individually selected rate into a powder production reactor having a first set of operating parameters, wherein each of the plurality of materials is a member of a set of possible constituent ingredients; and
ii. sampling at least a portion of a first output mixture of the powder production reactor, the first output mixture comprising one or more members of the set of constituent ingredients present in a first ingredient ratio; and
b. during a second production time:
i. introducing the plurality of materials into the powder production reactor having a second set of operating parameters, each at a second individually selected rate; and
ii. sampling at least a portion of a second output mixture of the powder production reactor, the second output mixture comprising one or more members of the set of constituent ingredients present in a second ingredient ratio.

5. The method of Claim 4, wherein the first and the second operating parameters are substantially the same.

6. The method of Claim 4, wherein the first and the second individually selected rates are substantially the same.

7. The method of Claim 4, wherein the second individually selected rates differ from the first individually selected rates only in the individually selected rate of a subset of the plurality of materials.

8. The method of Claim 4, further comprising repeating the step of introducing the plurality of materials, with a variety of sets of operating conditions, and the step of sampling at least a portion of an output mixture, to form a range of samples.

9. The method of Claim 4, wherein the duration of the first and second production times are long enough that a quasi-steady state is present in the first output mixture and the second output mixture.

10. The method of Claim 4, wherein the plurality of materials are provided one of the following forms: solid, liquid, and gas.

11. The method of Claim 4, wherein the powder production reactor is a plasma reactor.

12. The method of Claim 11, wherein the plasma reactor uses a plasma selected from one of the following types: constricted arc, free-burning electric arc, combustion flame, low pressure arc, and glow discharge.

13. The method of Claim 11, wherein the plasma reactor uses an energy delivery means selected from one of the following: high intensity (DC and AC), RF, inductively coupled, microwave, and a hybrid.

14. A process for high throughput production of a set of distinct materials from a group of precursor elements, comprising the steps of:
a. during a first production time:
i. introducing a first material input at a first selectable rate into a gas phase particle production reactor having a first set of operating parameters;
ii. introducing a second material input into the gas phase particle production reactor at a second selectable rate; and
iii. sampling at least a portion of an output mixture of the gas phase particle production reactor; and
b. repeating the steps of introducing a first material, introducing a second material, and sampling, during a second production time wherein a second set of operating parameters, a third selectable rate of introduction of the first material, and a fourth selectable rate of introduction of the second material are used.

15. The process of Claim 14, wherein the first set of operating parameters and the second set of operating parameters are substantially the same.

16. The process of Claim 14, wherein the first selectable rate and the third selectable rate are the same.

17. The process of Claim 14, wherein the second selectable rate and the fourth selectable rate are the same.

18. The process of Claim 14, further comprising repeating the steps of introducing through sampling during further production times to form a desired number of distinct output mixtures, wherein during each production time an independent set of operating parameters and two independent rates are used.

19. The process of Claim 14, wherein the set of operating parameters includes one or more of the following: first material, second material, relative location of introduction of the first and the second material, reactor energy, and reaction throughput rate.

20. The process of Claim 14, further comprising a step of introducing additional materials, each at an independent rate.

21. The process of Claim 14, wherein the first and second materials are provided one of the following forms: solid, liquid, and gas.

22. The process of Claim 14, wherein the gas phase particle production reactor is a plasma reactor using one of the following types of plasma: high intensity (DC and AC), RF, inductively coupled, microwave, and a hybrid.
